# EUROPEAN PATENT SPECIFICATION

(11) **EP 0 736 104 B1**
(45) Date of publication and mention of the grant of the patent: **16.01.2002**
(21) Application number: 94930699.7
(22) Date of filing: 11.10.1994
(51) Int. Cl.: C12Q 1/04, C12Q 1/00, C12Q 1/02, G01N 1/00, A61L 17/00

(54) **IMPROVED THREE REAGENT GRAM STAINING METHOD AND KIT**
DREI REAGENZIEN UMFASSENDES GRAM-FÄRBEVERFAHREN UND -KIT
COLORATION DE GRAM AMELIOREE A TROIS REACTIFS ET TROUSSE DE COLORATION

(30) Priority: 22.12.1993 US 172416
(43) Date of publication of application: 09.10.1996
(73) Proprietor: DIFCO LABORATORIES, Ann Arbor, Michigan 48108 (US)
(72) Inventor: MESZAROS, Amy, Taylor, Adrian, MI 49221 (US); STRENKOSKI, Leon, F., Dexter, MI 48130 (US)
(74) Representative: Davies, Gregory Mark
(86) International application number: PCT/US94/11503
(87) International publication number: WO 95/17519

(56) References cited:
- US-A- 4 225 669
- US-A- 4 857 459
- US-A- 4 916 061
- BIOLOGICAL ABSTRACTS, vol. 96, Philadelphia, PA, US; abstract no. 423228, XP002068357 & H. YUANTONG ET AL.: "A simplified Gam-stain and its quality control." WEISHENGWU XUEBAO, vol. 36, no. 1, 1996, CHINA, pages 76-78,
- JOURNAL OF CLINICAL MICROBIOLOGY, Volume 26, No. 7, issued July 1988, ROMERO et al., "Rapid Method for the Differentiation of Gram-Positive and Gram-Negative Bacteria on Membrane Filters", pages 1378-1382.

## Description

The present invention relates to a reagent and a staining method for bacteria. More particularly, the present invention relates to a Gram staining reagent combining a decolouriser and a counter-stain and a three reagent Gram staining method.

The Gram stain is a well known and widely used microbiological technique used for classifying and identifying bacteria. In hospitals and clinical laboratories, the Gram stain is one of the most frequently performed microbiological diagnostic tests. The Gram stain essentially divides the bacterial world into two distinct classifications: Gram positive bacteria and Gram negative bacteria. Separation into these two classes is based on differences in the structure of the bacterial walls.

The conventional and most commonly used method of the Gram staining technique requires the use of four separate reagents. The reagents consist of a primary staining solution usually containing crystal violet dye, a mordant solution usually containing an iodine-potassium iodide solution, a decolourising agent usually a solution of acetone and alcohol, and a counter-staining solution usually containing safranin. The conventional method involves the steps of: (1) treating a slide having bacteria affixed upon it with the principal stain, waiting 30-60 seconds; (2) rinsing the slide with water; (3) treating the slide with the mordant solution, waiting 30-60 seconds; (4) rinsing slide with water; (5) decolourising the slide with acetone and alcohol solution, waiting 30-60 seconds; (6) rinsing the slide with water; (7) counter-staining the slide with the safranin or fuchsin solution for 30-60 seconds and; (8) rinsing the slide with water. Only after these steps are completed can the bacteria affixed to the slide be observed and analysed to determine Gram status. Typically, Gram positive bacteria will appear purple in colour, while Gram negative bacteria will appear reddish-pink in colour.

It should be obvious from the above description of the conventional method of Gram staining that the conventional method of Gram staining is a very involved, time consuming, and labour intensive process. Each additional step introduces the possibility of error which can lead to an incorrect or inconclusive identification of the bacteria. A Gram stain which results in an inconclusive or incorrect identification of a bacterial specimen must either be repeated or have other diagnostic tests performed to more positively identify the bacterial specimen. In either case, the initiation of effective treatment is delayed when conclusive bacterial identification is not obtained.

For years, technicians using the conventional Gram staining method have been plagued by these inconsistent results. Much of the inconsistency is directly attributable to the conventional method itself. For instance, the separate decolourising step is problematic in that it is difficult to achieve the proper amount of decolourisation of the bacteria. In other words, decolourisation, the step of removing the fixed crystal violet stain from the Gram negative organisms, is prone to inconsistency. The inconsistency stems from difficulties associated with the amount of decolourising solvent applied to bacteria and the amount of time the decolourising solvent is allowed to contact the bacteria. If the decolourising solvent is applied in large volumes or is applied too vigorously, the bacteria in the sample may be under-stained, thereby potentially yielding an inconsistent or incorrect result i.e., a truly Gram positive bacterium may appear to be a Gram negative bacterium. If, on the other hand, a bacterial sample is not adequately decolourised, the bacteria in the sample may be over-stained, thereby potentially yielding an inconsistent or incorrect result, i.e., a truly Gram negative bacterium may appear to be a Gram positive bacterium.

Using the conventional four reagent Gram staining method, certain bacteria may appear as to be both Gram positive and Gram negative. These bacteria are referred to in the art as Gram variable bacteria. Gram variable bacteria present a unique challenge to the technician in that the results of a Gram stain on a pure culture of one of these bacteria could yield both Gram positive and Gram negative bacteria or the bacteria may be stained in such a way that it is impossible to make a determination of the bacteria's Gram status. Instead of Gram positive bacteria appearing purple in colour and Gram negative bacteria appearing reddish-pink in colour, the bacteria may appear brownish purple or simply just brown. A result like this makes identifying and classifying a bacterial sample very difficult, as is described below.

In addition to inconsistency, the conventional Gram staining method takes more time to perform, i.e., an additional reagent adds at least another one minute application period and an additional water wash. Also, the conventional Gram staining method is more costly, since costs typically increase as more reagents are involved and there is a cost associated with a technician or a machine performing the additional manipulations connected with an additional reagent.

U.S. Pat. No. 4,857,459 to Reuben discloses a method for staining acid-fast bacilli which utilises a decolourising counter-stain solution containing methylene blue, ethyl alcohol, potassium hydroxide, glycerol, acetic acid, and polyvinylpyrrolidone. However, the decolourising counter-stain solution and method for its use disclosed in the Reuben '459 patent are for performing an acid-fast stain and not for performing a Gram stain. Unlike the Gram stain, the acid-fast stain is dependent on the lipid containing cell walls of Mycobacteria and is intended for use primarily with these acid-fast bacilli.

There have been other attempts to streamline the conventional Gram staining method and reagents. U.S Patent No. 4,916,061 to Di Ianni discloses a Gram staining method in which the number of individual reagents was reduced by combining the mordant and the decolouriser into a single reagent comprised of an iodine-iodide salt complex and an alcohol solvent. The Di Ianni '061 patent sought to both streamline the Gram staining process and also to provide a more stable, longer lasting mordant solution. Unfortunately, this method provides inadequate results and, therefore, has no real utility, as described in detail below. It, therefore, follows that simply combining reagents from the conventional Gram staining method does not necessarily yield an improved method or unexpected results. Additionally, merely combining the counter-stain and the decolouriser without adjusting the pH to approximately 4.5 does not provide a pronounced improvement over prior art Gram staining methods.

The present invention not only streamlines the Gram staining method, it yields the added benefits of improved accuracy and performance by eliminating the certain long standing problems such as over/under decolourising and variability.

According to the present invention there is provided a method of Gram staining a specimen containing bacteria, which method comprises:
(a) staining Gram positive bacteria in the specimen;
(b) fixing the stain to the Gram positive bacteria; and
(c) simultaneously decolourising and counter-staining Gram negative bacteria in the specimen in a solution having a pH within the range of 1 to 6.

Other advantages of the present invention will be readily appreciated as the same becomes better understood by reference to the following detailed description when considered in connection with the accompanying drawings wherein:
FIG.1 is a photograph of *Bacillus megaterium* ATCC 14581, a Gram positive organism, which has been Gram stained using the traditional four-step method.
FIG.2 is a photograph of *Bacillus megaterium* ATCC 14581, a Gram positive organism, which has been Gram stained using the three-step method of the present invention.

The present invention provides a method for Gram staining a specimen containing bacteria and other cells. Prior to commencing the subject Gram staining method, a bacterial sample is first obtained and affixed to a support structure. The bacteria can be obtained from any suitable source such as a pure culture, a wound exudate, a blood smear, a sputum sample or the like. The bacteria are affixed to the support structure (which can be a standard glass microscope slide) by heating, alcohol treatment or other suitable means of fixation as are known in the art.

The first step in the subject method is staining the bacteria affixed on the slide with a reagent solution containing a primary dye capable of staining Gram positive bacteria. Crystal violet, or as it is sometimes referred to as gentian violet, is the preferred primary dye for staining Gram positive bacteria. The crystal violet dye is dissolved in a solvent which is typically water or an alcohol, such as ethanol or isopropanol. The crystal violet solution may also include certain other minor components, such as phenol or aniline, which are added to increase the solubility and stability of the crystal violet in the solution. Staining the bacteria affixed to the slide includes applying the crystal violet solution to the bacteria affixed to the slide using any suitable means such as a squeeze bottle, an eye dropper, or any other suitable means for applying a solution. The staining step also includes an application period in which the crystal violet solution is allowed to incubate or contact the bacteria for a period of time, typically one minute. Following the application period, the bacteria affixed to the slide are gently washed with water to remove any excess crystal violet solution.

Following the staining step, a mordant solution is applied to the bacteria in order to fix the primary dye within the bacteria. The mordant solution forms insoluble compounds with the primary dye, that is, the crystal violet dye present within the bacteria is altered so that it will only be retained by Gram positive bacteria. The mordant solution typically comprises an iodine-iodide salt complex dissolved in a solvent, such as water. The iodine comprising the iodine-iodide salt complex is generally crystallized iodine. The iodide salt can be any number of different iodide salts but is generally one of either potassium iodide, sodium iodide, magnesium iodide, aluminum iodide, zinc iodide, and ferrous iodide. Additionally, the mordant solution can comprise iodine complexed with polyvinylpyrrolidone. The mordant solution may also be comprised of an iodine concentrate/diluent preparation. The iodine concentrate/diluent preparation would provide a more stable means for transport and add to the shelf life of the solution.

Fixing the crystal violet dye within the Gram positive bacteria is accomplished by applying the mordant solution to the bacteria affixed to the slide using any suitable means such as a squeeze bottle, an eye dropper, or any other suitable means for applying a solution. The fixing step also includes an application period in which the mordant solution is allowed to incubate or contact the bacteria for a period of time, typically one minute.

Following the application period, the bacteria which were affixed to the slide and previously treated with the mordant solution are gently washed with a combination decolourising and counter-staining solution to remove any excess mordant solution. This final step in the subject Gram staining method simultaneously decolourises and counter-stains the previously treated bacteria affixed to the slide. The step of simultaneously decolourising and counter-staining the bacteria affixed to the slide allows for removal of any fixed crystal violet dye from Gram negative bacteria (decolourisation) which, because of the structure of their bacterial walls, do not retain fixed crystal violet dye and, at the same time, stains the bacteria with a second dye capable of staining Gram negative bacteria (counter-staining). In the final step of the subject method, a counter-stain is applied to the bacteria to stain the Gram negative bacteria with a differentiating stain to allow the Gram negative bacteria to be identified. In other words, a solution is applied to the bacteria which removes fixed crystal violet dye from any Gram negative bacteria present in bacterial specimen while, simultaneously, introducing a second stain into those same Gram negative bacteria to allow for the differentiation of both Gram positive and Gram negative bacteria.

The step of simultaneously decolourising and counter-staining Gram negative bacteria is accomplished by applying the combination decolourising and counter-staining solution to the bacteria affixed to the slide. The combination decolourising and counter-staining solution includes a solvent for removing the fixed crystal violet dye from Gram negative bacteria, a counter-stain for staining Gram negative bacteria, and a buffer solution.

In the preferred embodiment, the solvent is ethanol, but may be methanol, isopropanol, chloroform, or acetone. Additionally, water may be included in the solvent in concentrations ranging from about 0% to 25%, preferably about 10%.

The combination decolourising and counter-staining reagent solution also includes a stain capable of staining Gram negative bacteria. The stain can be any suitable counter-stain such as either fuchsin or safranin in concentrations ranging from 0.02% to 0.50% and 0.10% to 0.80%, respectively. In the preferred embodiment, the counter-stain is a mixture of both safranin and basic fuchsin, but any other suitable dye may be substituted as an additional counter-stain for use in combination with safranin such as acid fuchsin and neutral red dye. In the preferred embodiment, the concentration of safranin in the reagent solution for simultaneously decolourising and counter-staining the Gram negative bacteria can range from about 0.10% to 0.80%. The preferred concentration of safranin is about 0.40%. The concentration of the additional counter-stain, which in the preferred embodiment is basic fuchsin, can range from about 0.02% to 0.50%. The preferred concentration of the basic fuchsin is about 0.30%. The concentration of ethanol in the reagent solution for simultaneously decolourising and counter-staining the Gram negative bacteria can range from about 75% to 100%, and preferably is about 90%.

In order to enhance the staining quality of the specimen, a mordant can be added to the combination decolourising and counter-staining solution. The addition of a mordant at this step acts to enhance the staining results by fixing the counter-stains in those cells in which the counter-stains are taken up. That is, just as the mordant applied during the fixing step complexes with the Gram positive stain to prevent the stain from being washed from a Gram positive cell, the mordant applied during the simultaneous decolourising and counter-staining step functions to "fix" Gram negative stain within Gram negative cells.

Suitable mordants can include phenol, aniline, formalin, or similar mordants. In the preferred embodiment, the mordant is phenol. The concentration of the mordant can range from 1% to 10%; however, in the preferred embodiment the concentration of the mordant, i.e., phenol, is-approximately 5%.

In addition to the constituents which comprise the combination decolourising and counter-staining solution, the pH of the solution is also critical to the present invention. The optimal pH range for the combination decolourising and counter-staining solution is in the acidic range between pH=1 to pH=6. The preferred pH for the solution is approximately pH=4.5. Acidification is critical in order to resolve and differentiate mammalian cells which may be present in a clinical specimen. Without adjusting the pH of the combination decolourising and counter-staining solution within this range, mammalian cells such as polymorphonuclear leukocytes (PMN's), mononuclear cells, epithelial cells and similar type cells are difficult to distinguish and resolve from a Gram stained bacterial slide. These other cells can be a valuable aid in diagnosis and bacterial typing since they are important indicators of inflammation and specimen quality. Manipulation of pH of the solution directly effects Gram stain results of bacteria and mammalian cells. Without the proper pH, the bacteria over-decolourise and the mammalian cells do not stain at all. The combination decolourising and counter-staining solution of the present invention can also be used in order to specifically stain mammalian cells, such as polymorphonuclear leukocytes, in a specimen or sample which may or may not additionally contain bacteria. That is, at this critical pH mammalian cells can be stained and, therefore, visualised. In other words, the reagents and steps of the present Gram staining method can be used to not only visualise and identify bacterial cells present in a specimen or sample but, additionally, to stain, visualise, and identify mammalian cells present in a specimen or sample.

The pH of the combination decolourising and counter-staining solution can be adjusted in order to compensate for pH variances caused by the particular formulation of the solution. That is, depending on the constituents comprising the combination decolourising and counter-staining solution, the pH may be required to be adjusted up (add base) or down (add acid) in order to obtain the optimum pH, i.e., approximately pH 4.5. The pH of the solution can be lowered (acidified) by the addition of acids such as glacial acetic acid or hydrochloric acid. The pH of the solution can be raised (made more basic) by adding bases such as sodium hydroxide.

Another method of adjusting the pH of the solution is by buffering. The buffer in the combination decolourising and counter-staining solution may be any number of different buffering agents but is generally acetate, TRIS, or other suitable buffers known to those skilled in the art. Many buffers may be suitable as long as they are soluble in the solution and possess similar buffering capacity in the preferred pH range. The preferred buffer is an acetate buffer ranging in concentration from 0.01M to 1M. The preferred concentration of acetate buffer is 0.1M. Buffering the combination decolourising and counter-staining solution is crucial for staining mammalian cells present in specimens to be analysed.

The simultaneous decolourising and counter-staining step is accomplished by rinsing the mordant from the slide using the combination decolourising and counter-staining solution and then applying the combination decolourising and counter-staining solution to the bacteria affixed to the slide using any suitable means such as a squeeze bottle, an eye dropper, or any other suitable means for applying a solution. Additionally, the use of the simultaneous decolourising and counter-staining step eliminates a water washing step from the conventional Gram staining method. The simultaneous decolourising and counter-staining step also includes an application period in which the combination decolourising and counter-staining solution is allowed to incubate or contact the bacteria for a period of time, typically 20-50 seconds. Following the application period, the bacteria affixed to the slide are gently washed with water to remove any excess decolourising and counter-staining solution. At this point, the bacteria affixed to the slide are in a condition suitable for microscopic analysis to determine if the bacteria are Gram positive, Gram negative, or a combination of both.

The present invention also includes a kit containing the Gram staining reagents including a first reagent means for staining Gram positive bacteria; a second reagent means for fixing the stain to the Gram positive bacteria; and a third reagent means for simultaneously decolourising and counter-staining Gram negative bacteria, the third reagents means having a pH within the range of 1 to 6.

The third reagent means preferably comprises a solution consisting of at least one counter-stain (preferably one or more of safranin, basic fushin and natural red stain) and a decolourising solvent. The solution is typically pH adjusted, such that the pH of the solution is within the range of 1 to 6.

The present invention also includes a Gram staining reagent for simultaneously decolourising and counter-staining bacteria affixed to a slide. The reagent comprises a decolourising solvent and at least one counter-stain and is pH adjusted such that the pH of the reagent is within the range of 1 to 6.

### Example 1

### Materials and Methods

Samples of microorganisms including bacterial and yeast specimens (see Table 1) were affixed to glass microscope slides and were treated with conventional four reagent Gram staining method comprised of an aqueous principal staining solution containing 0.38% crystal violet, 0.44% phenol; a mordant solution containing 0.33% iodine crystals, 0.66% potassium iodide; a decolourising solution containing acetone and isopropanol in a ratio of 1:3 respectively; and a counter-staining solution containing 0.4% safranin and 20% ethanol.

Using the conventional Gram staining method the microorganisms were: (1) treated with the principal staining solution for 30-60 seconds; (2) rinsed with water; (3) treated with the mordant solution for 30-60 seconds; (4) rinsed with water; (5) treated with the decolourising solution for 30-60 seconds; (6) rinsed with water; (7) treated with the counter-staining solution for 30-60 seconds and; (8) rinsed with water. Following these steps, the microorganisms were microscopically analysed and the results are shown in Table 1.

For the three reagent method of the present invention, identical microorganism samples, including bacterial and yeast specimens, were affixed to glass microscope slides and were treated with a principal staining solution containing 0.38% crystal violet, 0.44% phenol; a mordant solution containing 10% polyvinylpyrrolidone-iodine, 1.9% potassium iodide; and a combination decolourising and counter-staining solution containing 0.40% safranin, 90% ethanol, and 10% water in a 0.1M acetate buffer, pH 4.5. Using the three reagent Gram staining method of the present invention, the microorganism samples were: (1) treated with the principal staining solution for 30-60 seconds; (2) rinsed with water; (3) treated with the mordant solution for 30-60 seconds; (4) rinsed with the combination decolourising and counter-staining solution; (5) treated with the combination decolourising and counter-staining solution for 20-50 seconds; and (6) rinsed with water. Following these steps, the microorganism samples were microscopically analysed and the results are shown in Table 1.

### Results

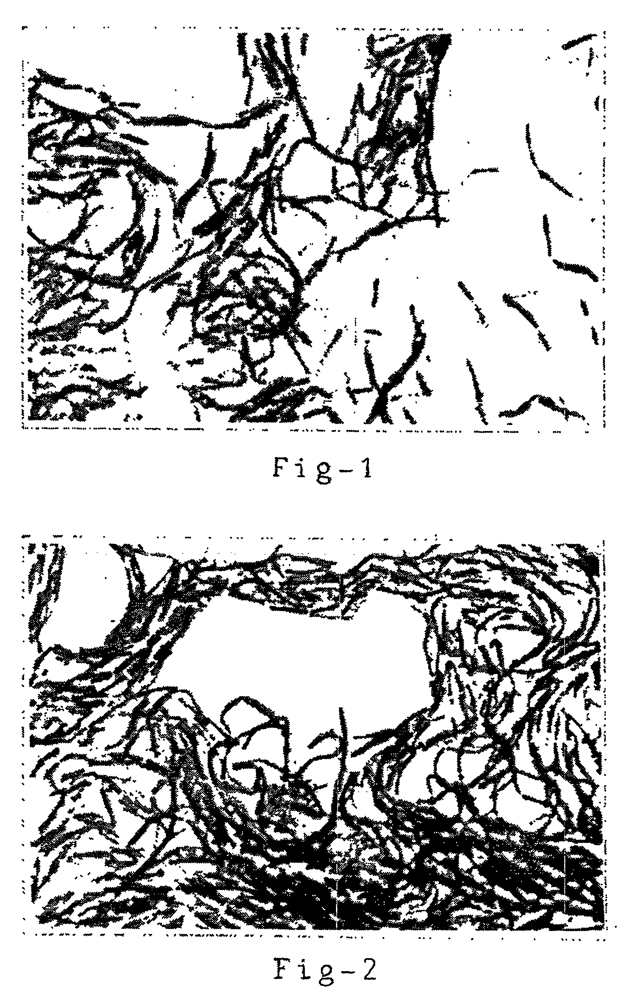

Referring to Figs. 1 and 2, tests were performed comparing the three reagent Gram staining method of the present invention with the conventional four reagent Gram staining method. Applicants found that for certain Bacillus sp. the subject reagent and method yielded consistent, easily interpreted Gram stain results, while the conventional Gram staining reagents and method yielded inconclusive results. Referring to Fig. 1, the bacteria Gram stained by the traditional four step method appear to be yellowish in colour which is inconsistent with the purple colour which indicates Gram positive organisms and the pinkish colour which indicates Gram negative organisms. However, referring to Fig. 2, the bacteria Gram stained by the three step method of the subject invention are purple in colour, clearly indicating that the organism is Gram positive.

### Example 2.

### Materials and Methods

For the three reagent method of the present invention, clinical samples were affixed to glass microscope slides and were treated with a principal staining solution containing 0.38% crystal violet, 0.44% phenol; a mordant solution containing 10% polyvinylpyrrolidone-iodine crystals, 1.9% potassium iodide; and a combination decolourising and counter-staining solution containing 0.40% safranin, 90% ethanol, and 10% water in a 0.1M acetate buffer, pH 4.5.

Using the three reagent Gram staining method of the present invention, the microorganism samples were: (1) treated with the principal staining solution for 30-60 seconds; (2) rinsed with water; (3) treated with the mordant solution for 30-60 seconds; (4) rinsed with the combination decolourising and counter-staining solution; (5) treated with the combination decolourising and counter-staining solution for 20-40 seconds; (6) rinsed with water. Following these steps, the microorganism samples were microscopically analysed and the results are shown in Table 2.

### Results

Tests were performed on clinical samples using the three reagent Gram staining method of the present invention. Applicants found that the three reagent method of the present invention yielded consistent and easily interpreted Gram staining results for a variety of clinically isolated samples. The three reagent method provided accurate, consistent, and easily interpretable results for a variety of typical clinical isolates. These results illustrate the benefits obtained by adjusting the pH of the combination decolourising and counter-staining solution. Additionally, these results demonstrate the criticality of buffering and acidifying the pH of the combination decolourising and counter-staining solution in order to stain mammalian cells present in the samples. Without adjusting the pH and buffering the solution, the mammalian cells are not sufficiently differentiable and, therefore, cannot be used to aid in identification or diagnosis. Polymorphonuclear leukocytes (PMN's), epithelial cells and similar type cells were easily distinguishable using the Gram staining method of the present invention and aided in the interpretation of the clinical samples.

### Example 3.

### Material and Methods

Samples of microorganisms including bacterial and yeast specimens (see Table 3) were affixed to glass microscope slides and were treated with three reagent Gram staining method of Di Ianni comprised of a principal staining solution containing 1.2 gram crystal violet dye, 2 grams of aniline, and 10% (V/V) of isopropanol in a 100 ml final volume; a combination mordant and decolourising solution containing 0.66% potassium iodide, 0.33% iodine crystals, and 95% ethanol; and a counter-staining solution containing 1% safranin in an aqueous solution.

Using the three reagent Gram staining method of the Di Ianni '061 patent, microorganism samples were: (1) treated with the principal staining solution for one minute; (2) rinsed with water; (3) treated with the combination mordant and decolourising solution for one minute; (4) rinsed with water; (5) treated with the counter-staining solution for 30-60 seconds; and (6) rinsed with water. Following these steps, the microorganism samples were microscopically analysed and the results are shown in Table 3.

For the three reagent method of the present invention, identical bacterial specimens were affixed to glass microscope slides and were treated with a principal staining solution containing 0.38% crystal violet, 0.44% phenol; a mordant solution containing 10% polyvinylpyrrolidone-iodine, 1.9% potassium iodide; and a combination decolourising and counter-staining solution containing 0.40% safranin, 90% ethanol, and 10% water in a 0.1M acetate buffer as previously described in Example 1.

### Results

Tests were performed comparing the three reagent Gram staining method of the present invention with the three reagent Gram staining method of the Di Ianni '061 patent described above. Applicants found that for organisms from both Gram positive and Gram negative groups, the combination decolourising and counter-staining reagent and method of the present invention yielded consistent and easily interpreted Gram staining results, while the three reagent Gram staining reagent and method of the Di Ianni '061 patent yielded inconclusive and sometimes incorrect results. For example, for Staphyloccus aureus ATCC 2048, Staphyloccus xylosis ATCC 29971, Bacillus cereus ATCC E-14579, Bacillus megaterium ATCC 14581, and Bacillus subtilis ATCC 11774, the Di Ianni reagent and method yielded either incorrect Gram determinations or inconclusive Gram determinations, while the three reagent method of the present invention yielded consistently accurate and easily interpreted results for the same organisms.

### Example 4.

### Materials and Methods

Samples of microorganisms including clinical samples containing bacterial and yeast specimens (see Table 4) were affixed to glass microscope slides and were treated with conventional four reagent Gram staining method comprised of an aqueous principal staining solution containing 0.40% crystal violet; a mordant solution containing 13% polyvinylpyrrolidone-iodine complex, 1.9% potassium iodide; a decolourising solution containing acetone and isopropanol in a ratio of 1:3, respectively; and a counter-staining solution containing 0.25% safranin and 20% ethanol.

Using an automatic staining device (EMDS Midas II, EM Science, Gibbstown, NJ) to apply the conventional Gram staining method the microorganisms were: (1) treated with the principal staining solution for 10 seconds; (2) rinsed with water; (3) treated with the mordant solution for 10 seconds; (4) rinsed with water; (5) treated with the decolourising solution for 7 seconds; (6) rinsed with water; (7) treated with the counter-staining solution for 10 seconds and; (8) rinsed with water. Following these steps, the microorganisms were microscopically analysed and the results are shown in Table 4.

For the three reagent method of the present invention, identical samples of microorganisms including clinical samples containing bacterial and yeast specimens were affixed to glass microscope slides and were treated with a principal staining solution containing 0.38% crystal violet, 0.44% phenol; a mordant solution containing 10% polyvinylpyrrolidone-iodine, 1.9% potassium iodide; and a combination decolourising and counter-staining solution containing 0.40% safranin, 0.30% basic fuchsin, 90% ethanol, 10% water, and acidified to pH 4.5. Using the three reagent Gram staining method of the present invention, the microorganism samples were: (1) treated with the principal staining solution for 30-60 seconds; (2) rinsed with water; (3) treated with the mordant solution for 30-60 seconds; (4) rinsed with the combination decolourising and counter-staining solution; (5) treated with the combination decolourising and counter-staining solution for 20-50 seconds; and (6) rinsed with water. Following these steps, the microorganism samples were microscopically analysed and the results are shown in Table 4.

### Results

Referring to Table 4, tests were performed comparing the three reagent Gram staining method of the present invention with the conventional four reagent Gram staining method. Applicants found for bacterial and yeast samples that the 3-step method of the present invention was comparable to the standard 4-step method. However, applicants data shows that for non-bacterial cells (mammalian cells, immune system cells) that in some cases, the 3-step method of the present invention yielded superior results in the staining and visualisation of non-bacterial cells than did the standard 4-step method.

### Example 5.

### Material and Methods

Clinical samples containing microorganisms (see Table 5) were affixed to glass microscope slides and were treated with conventional four reagent Gram staining method comprised of an aqueous principal staining solution containing 0.38% crystal violet, 0.44% phenol; a mordant solution containing 0.33% iodine crystals, 0.66% potassium iodide; a decolourising solution containing acetone and ethyl alcohol in a ratio of 1:1, respectively; and a counter-staining solution containing 0.4% safranin and 20% ethanol.

Using the conventional Gram staining method the microorganisms were: (1) treated with the principal staining solution for 60 seconds; (2) rinsed with water; (3) treated with the mordant solution for 60 seconds; (4) rinsed with water; (5) treated with the decolourising solution until no more color ran from the slide; (6) rinsed with water; (7) treated with the counter-staining solution for 60 seconds and; (8) rinsed with water. Following these steps, the microorganisms were microscopically analysed and the results are shown in Table 4.

For the three reagent method of the present invention, identical clinical samples containing microorganisms were affixed to glass microscope slides and were treated with a principal staining solution containing 0.38% crystal violet, 0.44% phenol; a mordant solution containing 0.33% iodine crystals, 0.66% potassium iodide; and a combination decolourising and counter-staining solution containing 0.38% safranin, 0.28% basic fuchsin, 90% ethanol, 5% phenol, 10% water, and acidified to pH 4.5. Using the three reagent Gram staining method of the present invention, the microorganism samples were: (1) treated with the principal staining solution for 30-60 seconds; (2) rinsed with water; (3) treated with the mordant solution for 30-60 seconds; (4) rinsed with the combination decolourising and counter-staining solution; (5) treated with the combination decolourising and counter-staining solution for 20-50 seconds; and (6) rinsed with water. Following these steps, the microorganism samples were microscopically analysed and the results are shown in Table 5.

### Results

Referring to Table 5, tests were performed comparing the three reagent Gram staining method of the present invention with the conventional four reagent Gram staining method. Applicants found that the 3-step method utilising the combination decolourising and counter-staining solution was superior to the standard 4-step Gram staining method in discerning both microbiological as well as mammalian cells from an actual human specimen. The results depicted in Table 5 illustrate that not only does the 3-step method and combination decolourising and counter-staining solution of the present invention yield superior results over the standard in identification and classification of bacterial cells, the 3-step method and combination decolourising and counter-staining solution of the present invention yields far superior results in. the staining and identification of mammalian cells such as polymorphonuclear leukocytes.

The invention has been described in an illustrative manner, and it is to be understood that the terminology which has been used is intended to be in the nature of words of description rather than of limitation.

Obviously, many modifications and variations of the present invention are possible in light of the above teachings. It is, therefore, to be understood that within the scope of the appended claims, the invention may be practiced otherwise than as specifically described.

**TABLE 1**

| **Microorganism** | **Conventional Four Reagent Gram Staining Method** | **Three Reagent Gram Staining Method of the Present Invention** |
|---|---|---|
| Corynebacterium xerosis ATCC 9016 | g⁺ | g⁺ |
| Pseudomonas aeruginosa ATCC 10145 | g⁻ | g⁻ |
| Candida albicans ATCC 10231 | g⁺ | g⁺ |
| Candida tropicalis ATCC 11307 | g⁺ | g⁺ |
| Staphylococcus aureus ATCC 25923 | g⁺ | g⁺ |
| Staphylococcus aureus CDC 2048 | g⁺ | g⁺ |
| Staphylococcus xylosis ATCC 29971 | g⁺ | g⁺ |
| Micrococcus sp. IMC 72235 | g⁺ | g⁺ |
| Klebsiella pneumoniae BMC 3292 | g⁻ | g⁻ |
| Klebsiella pneumoniae ATCC 13883 | g⁻ | g⁻ |
| Klebsiella oxytoca ATCC 8724-A | g⁻ | g⁻ |
| Escherichia coli CDC 313-83 | g⁻ | g⁻ |
| Bacillus cereus ATCC 14579 | g⁺ | g⁺ |
| Bacillus megaterium ATCC 14581 | g⁺ | g⁺ |
| Bacillus subtilis ATCC 6633 | g⁺ (purple, brownish) | g⁺ (all purple) |
| Bacillus subtilis ATCC 11774 | g⁺,g⁻ (brownish) | g⁺ |

**TABLE 2**

| **Specimen** | **Result: 3-Reagent Method** |
|---|---|
| Blood A | g⁺, cocci |
| Blood B | g⁻, rod; PMN's |
| Blood C | g⁻, rod |
| Blood D | g⁺, cocci; PMN's |
| Wound A | g⁺, cocci; |
| | g-, diplococci (bipolar bacilli) |
| Wound B | g⁺, cocci |
| Sputum A | g⁺, cocci; g⁻ rods; PMN's |
| Vaginal A | epithelial cells |
| Throat A | g⁺, coccobacilli; squamous cells |

**TABLE 3**

| **Microorganism** | **Di Ianni '061 Three Step Gram Staining Method** | **Three Step Gram Staining Method of the Present Invention** |
|---|---|---|
| Staphyloccus aureus ATCC 2048 | g- | g+ |
| Staphyloccus xylosis ATCC 29971 | pink-brown | g+ |
| Bacillus cereus ATCC E-14579 | g⁺,g⁻ | g⁺ |
| Bacillus megaterium ATCC 14581 | g⁺,g⁻ | g⁺ |
| Klebsiella pneumoniae BMC 3292 | g⁻ | g⁻ |
| Escherichia coli CDC 313-83 | g⁻ | g⁻ |
| Bacillus subtilis ATCC 6633 | g⁺ | g⁺ |
| Bacillus subtilis ATCC 11774 | g⁻ | g⁺ |

**TABLE 4**

| | **Bacterial Gram Stain/Morphology** | | **Non-Bacterial Staining Characteristics** | | |
|---|---|---|---|---|---|
| **Type of Specimen or Culture** | **Standard 4-Step** | **3-Step** | **Standard 4-Step** | **3-Step** | **Comments** |
| N. gonorrhoeae | Comparable | Comparable | Comparable | Comparable | Background darker on 3-Step |
| H. influenzae | Comparable | Comparable | Comparable | Comparable | 3-Step more intense |
| S. aureus | Comparable | Comparable | Comparable | Comparable | Equivalent |
| Vaginal 1 | S. epidermidis | S. epidermidis | Comparable | Comparable | Equivalent |
| Vaginal 2 | S. epidermidis Bacillus | S. epidermidis Bacillus | Comparable | Comparable | Equivalent |
| Sputum 3 | S. epidermidis mixed | S. epidermidis mixed | Comparable | Comparable | 3-Step more intense |
| Sputum 2 | PMN, Cocci Yeast | PMN, Cocci Yeast | Comparable | Comparable | Equivalent |
| Blood 1 | G+ bacillus | G+ bacillus | Comparable | Comparable | Equivalent |
| Blood 2 | G-bacillus | G-bacillus | Comparable | Comparable | Equivalent |

**TABLE 5**

| | **Bacterial Gram Stain/Morphology** | | **Non-Bacterial Staining Characteristics** | | |
|---|---|---|---|---|---|
| **Type of Specimen or Culture** | **Standard 4-Step** | **3-Step** | **Standard 4-Step** | **3-Step** | **Comments** |
| Fecal leukocyte | Gram negative bacilli hard to see GPB O.K. | Bacteria stand out | Faint pink | PMN's stand out | 3-Step better for PMN's & bacteria "clear smear" |
| Fecal leukocyte | No bacteria Seen | Gram negative bacilli stand out | Light pink PMN's | Darker pink PMN's | PMN's standout in 3-step, missed Gram negative bacilli in standard |
| Urine | Gram positive cocci O.K. Gram negative bacilli light | Gram positive cocci and Gram negative bacilli good | PMN's light | PMN's good | 3-Step enhanced Gram negative bacilli and PMN's good |
| CSF | Gram positive cocci | Gram positive cocci | PMN's good | PMN's good | No Difference |
| CSF | large dividing Gram positive cocci | large dividing Gram positive cocci | PMN light | PMN excellent | Cellular (PMN's) stained better in 3-Step. Easier to identify |
| Bone | Rare Gram positive cocci | Rare Gram positive cocci | Light PMN | PMN's good | Cells & bacteria easier to see in 3-Step, especially since they were rare. |

## Claims

1. A method of Gram staining a specimen containing bacteria, which method comprises:
(a) staining Gram positive bacteria in the specimen;
(b) fixing the stain to the Gram positive bacteria; and
(c) simultaneously decolourising and counter-staining Gram negative bacteria in the specimen in a solution having a pH within the range of 1 to 6.

2. A method according to claim 1, wherein said solution of step (c) comprises a decolourising solvent and at least one counter-stain, said solution being pH adjusted, such that the pH of the solution is within said range.

3. A method according to claim 2, wherein said solution includes a mordant.

4. A method according to claim 2 or 3, wherein said counter-stain comprises one or more of safranin, basic fuchsin, and neutral red stain.

5. A method according to claim 2 or 3, wherein said counter-stain comprises a mixture of safranin and an additional counter-stain selected from basic fuchsin, or neutral red dye.

6. A method according to claim 1 wherein said solution of step (c) comprises a decolourising solvent and a counter-stain, said counter-stain comprising a mixture of at least one counter-stain and an additional counter-stain, the counter-stain and the additional counter-stain being selected from safranin, basic fuchsin, and neutral red dye.

7. A kit for Gram staining containing reagents comprising:
first reagent means for staining Gram positive bacteria;
second reagent means for fixing the stain to the Gram positive bacteria; and
third reagent means for simultaneously decolourising and counter-staining Gram negative bacteria, said third reagent means having a pH within the range of 1 to 6.

8. A kit according to claim 7, wherein said third reagent means comprises a solution consisting of at least one counter-stain and a decolourising solvent, the solution being pH adjusted, such that the pH of the solution is within said range.

9. A kit according to claim 8, wherein said counter-stain comprises one or more of safranin, basic fuchsin, and neutral red stain.

10. A Gram staining reagent for simultaneously decolourising and counter-staining bacteria affixed to a slide, said reagent comprising a decolourising solvent and at least one counter-stain, the reagent being pH adjusted such that the pH of the reagent is within the range of 1 to 6.

## Patentansprüche

1. Verfahren zum Gram-Färben einer bakterienhaltigen Probe, umfassend die Schritte:
(a) Färbung der Gram-positiven Bakterien in der Probe;
(b) Fixierung der Farbe auf den Gram-positiven Bakterien; und
(c) gleichzeitige Entfärbung und Kontrast-Färbung der Gram-negativen Bakterien in der Probe in einer Lösung, die einen pH-Wert innerhalb des Bereichs von 1 bis 6 aufweist.

2. Verfahren nach Anspruch 1, bei dem die Lösung des Schrittes (c) ein Entfärbungsmittel enthält und mindestens eine Kontrastfarbe, wobei der pH-Wert der Lösung so eingestellt ist, daß er innerhalb des besagten Bereichs liegt.

3. Verfahren nach Anspruch 2, bei dem die besagte Lösung ein Beizmittel enthält.

4. Verfahren nach Anspruch 2 oder 3, bei dem die Kontrastfarbe eine oder mehrere der Farben Safranin, Basic-Fuchsin und Neutralrot enthält.

5. Verfahren nach Anspruch 2 oder 3, bei dem die Kontrastfarbe eine Mischung von Safranin und einer zusätzlichen, aus Basic-Fuchsin oder Neutralrot ausgewählten Kontrastfarbe enthält.

6. Verfahren nach Anspruch 1, bei dem die Lösung des Schrittes (c) ein Entfärbungsmittel und eine Kontrastfarbe enthält, wobei die Kontrastfarbe eine Mischung von mindestens einer Kontrastfarbe und einer zusätzlichen Kontrastfarbe umfaßt und die Kontrastfarbe und die zusätzliche Kontrastfarbe ausgewählt sind aus Safranin, Basic-Fuchsin und Neutralrot.

7. Gram-färbende Reagenzien enthaltendes Kit, umfassend erste Reagenzmittel zum Färben Gram-positiver Bakterien, zweite Reagenzmittel zum Fixieren der Farbe auf den Gram-positiven Bakterien und dritte Reagenzmittel, um gleichzeitig die Gram-negativen Bakterien zu entfärben und mit einer Kontrastfarbe zu versehen, wobei die dritten Reagenzmittel einen pH-Wert innerhalb des Bereichs von 1 bis 6 aufweisen.

8. Kit nach Anspruch 7, bei dem die dritten Reagenzmittel eine Lösung aus mindestens einer Kontrastfarbe und einem Entfärbungsmittel enthalten und der pH-Wert der Lösung so einjustiert ist, daß er innerhalb des besagten Bereichs liegt.

9. Kit nach Anspruch 8, bei dem die Kontrastfarbe eine oder mehrere der Farben Safranin, Basic-Fuchsin und Neutralrot umfaßt.

10. Gram-Färbereagenz zum gleichzeitigen Entfärben und Kontrastfärben von Bakterien, die auf einem Objektträger fixiert sind, wobei das Reagenz ein Entfärbungsmittel und mindestens eine Kontrastfarbe enthält und sein pH-Wert so justiert ist, daß er innerhalb des Bereichs von 1 bis 6 liegt.

## Revendications

1. Procédé de coloration de Gram d'un échantillon contenant des bactéries, laquelle coloration comprend :
(a) la coloration de Gram positive dans l'échantillon le spécimen ;
(b) la fixation de la coloration des bactéries de Gram positives ; et
(c) la décoloration et la contre coloration simultanée des bactéries Gram négatives dans l'échantillon dans une solution ayant un pH compris dans la plage allant de 1 à 6.

2. Procédé selon la revendication 1, dans lequel ladite solution de l'étape (c) comprend un solvant décolorant et au moins un contre colorant, le pH de ladite solution étant ajusté, de telle sorte que le pH de la solution soit compris dans ladite plage.

3. Procédé selon la revendication 2, dans lequel ladite solution comprend un mordant.

4. Procédé selon la revendication 2 ou 3, dans lequel ledit contre colorant comprend un ou plusieurs colorants safranine, fuchsine basique, et rouge neutre.

5. Procédé selon la revendication 2 ou 3, dans lequel ledit contre colorant comprend un mélange de safranine et un contre colorant supplémentaire choisie parmi la fuchsine basique ou le rouge neutre.

6. Procédé selon la revendication 1, dans lequel ladite solution de l'étape (c) comprend un solvant décolorant et un contre colorant, ledit contre colorant comprenant un mélange d'au moins un contre colorant et un contre colorant supplémentaire, le contre colorant et le contre colorant supplémentaire étant choisis parmi la safranine, la fuchsine basique et le rouge neutre.

7. Ensemble pour la coloration de Gram contenant des réactifs comprenant :
des premiers moyens réactifs pour la coloration des bactéries Gram positives ;
des deuxièmes moyens réactifs pour fixer la coloration sur les bactéries Gram positives ; et
des troisièmes moyens réactifs pour la décoloration et la contre coloration simultanées des bactéries Gram négatives, lesdits troisièmes moyens réactifs ayant un pH compris dans la plage allant de 1 à 6.

8. Ensemble selon la revendication 7, dans lequel lesdits troisièmes moyens réactifs comprennent une solution comprenant au moins un contre colorant et un solvant décolorant, le pH de la solution étant ajusté de telle sorte que le pH de la solution soit compris dans ladite plage.

9. Ensemble selon la revendication 8, dans lequel ledit contre colorant comprend un ou plusieurs colorant safranine, fuchsine basique, et rouge neutre.

10. Réactif de coloration de Gram pour la décoloration et la contre coloration simultanée des bactéries, collé sur une lamelle de verre, ledit réactif comprenant un solvant décolorant et au moins un contre colorant, le pH du réactif étant ajusté de telle sorte que le pH du réactif soit compris dans la plage allant de 1 à 6.
